# EUROPEAN PATENT SPECIFICATION

(11) **EP 2 990 035 B1**
(45) Date of publication and mention of the grant of the patent: **07.03.2018**
(21) Application number: 14786113.2
(22) Date of filing: 26.03.2014
(51) Int. Cl.: A61K 31/351, A61K 8/60, A61P 17/00, A61P 43/00, A61Q 1/02, A61Q 7/00, A61Q 19/00, A61Q 19/08, A61Q 19/10, A23L 2/52

(54) **1,5-ANHYDRO-D-GLUCITOL-CONTAINING COLLAGEN PRODUCTION ACCELERATOR**
1,5-ANHYDRID-D-GLUCITOL-HALTIGER BESCHLEUNIGER DER KOLLAGENPRODUKTION
ACCÉLÉRATEUR DE PRODUCTION DE COLLAGÈNE CONTENANT DU 1,5-ANHYDRO-D-GLUCITOL

(30) Priority: 15.04.2013 JP 2013084534
(43) Date of publication of application: 02.03.2016
(73) Proprietor: National University Corporation University Of Toyama, Toyama-shi, Toyama 930-8555 (JP); Fushimi Pharmaceutical Co., Ltd., Marugame-shi, Kagawa 763-8605 (JP)
(72) Inventor: KATO, Atsushi, Toyama-shi, Toyama 930-0194 (JP); ISHIKAWA, Fumihiro, Marugame-shi, Kagawa 763-8605 (JP); TAKESHITA, Kei, Marugame-shi, Kagawa 763-8605 (JP)
(74) Representative: Vossius & Partner Patentanwälte Rechtsanwälte mbB
(86) International application number: PCT/JP2014/058542
(87) International publication number: WO 2014/171279

(56) References cited:
- WO-A1-2010/082661
- WO-A2-2012/097052
- JP-A- 2008 054 531
- UA-C2- 72 003

## Description

### TECHNICAL FIELD

The present invention relates to a collagen production promoter containing 1,5-anhydro-D-glucitol (which may be simply referred to herein as "1,5-AG") or a derivative thereof, and a composition containing the same.

### BACKGROUND ART

Skin is constructed by three kinds of layered structures roughly divided into epidermis, dermis, and subcutaneous tissues. The epidermis is composed of a corneal layer contacting the outside and a basal layer generating new skin cells, and the dermis plays a role in supporting the basal layer. Representative ingredients for this dermal part include collagen, elastin, and hyaluronic acid, and especially 70% of the dermal part is made of collagen, the collagen production playing a key role in preventing and improving wrinkles.

For example, as a composition promoting collagen production, Patent Publication 1 discloses a cosmetic composition containing a plant extract containing soybean seeds, soybean germs, soybean embryos, soybean buds, maize (wheat) seeds; wheat germs, wheat embryos, soy-milk, tofu wastes (*okara*), or the like. Patent Publication 2 discloses a moisturizing agent containing an acyl oligopeptide, proline, and hydroxyproline, or the like.

On the other hand, sugar alcohols have been known to have moisturizing effects. For example, Patent Publication 3 discloses an external composition containing sorbitol, mannitol, xylitol, or glycerol. Patent Publication 4 discloses cosmetics containing sorbitol, maltitol, xylitol, or erythritol.

WO 2010/082661 discloses a composition for ameliorating postcibal hyperglycemia comprising at least one member from the group consisting of 1,5-D-anhydroglucitol, 1,5-D-anhydrofructose and derivatives thereof.

### PRIOR ART REFERENCES

### PATENT PUBLICATIONS

Patent Publication 1: Japanese Patent Laid-Open No. 2009-234944
Patent Publication 2: Japanese Patent Laid-Open No. 2008-195651
Patent Publication 3: Japanese Patent Laid-Open No. 2008-247752
Patent Publication 4: Japanese Patent Laid-Open No. 2004-059473

### SUMMARY OF THE INVENTION

### PROBLEMS TO BE SOLVED BY THE INVENTION

However, many of the conventional materials having collagen production promoting functions are extract compositions from plants, of which biocompatibility has not been clarified or insufficient, and of which chemical structures have been indefinite or undesirably degraded by reactions with other ingredients in cosmetics in many cases. In addition, sugar alcohols having collagen production promoting functions have not been known, and moisturizing agents containing a conventionally used sugar alcohol as a moisturizing main ingredient have been found to be unsatisfactory in the feel of use in many cases.

The present invention discloses a collagen production promoter having excellent biocompatibility and feel of use, and a composition containing the collagen production promoter. The present invention provides the formulation, non-therapeutic methods for promoting collagen production in cells, preventing and/or improving wrinkles of skin and the non-therapeutic uses as claimed in claims 1 to 5 attached.

### MEANS TO SOLVE THE PROBLEMS

Since the 1,5-AG is a non-reducing sugar in which 1-position of the D-glucose is reduced, its reactivity is lower than the reducing sugars, so that it is chemically stable under acidic, alkaline, high-temperature and other conditions. In addition, the 1,5-AG is second to most abundant sugars after D-glucose in a body, and it has excellent properties of having high biocompatibility and being free of problems in safety.

Therefore, the present inventors have remarked on 1,5-AG and made intensive studies, and as a result, they have found that the 1,5-AG can be utilized as a novel material having collagen production promoting ability. The present invention has been perfected thereby.

Specifically, the present specification discloses the following [1] to [10]:
[1] A collagen production promoter in cells, containing at least one member selected from the group consisting of 1,5-anhydro-D-glucitol and derivatives thereof.
[2] A composition containing the collagen production promoter as defined in the above [1].
[3] A method for promoting collagen production in cells, including the step of administering at least one member selected from the group consisting of 1,5-anhydro-D-glucitol and derivatives thereof to an individual in need of collagen production promotion in the cells in an effective amount.
[4] A method for preventing and/or improving wrinkles of skin, including the step of administering at least one member selected from the group consisting of 1,5-anhydro-D-glucitol and derivatives thereof to an individual in need of prevention and/or improvement of wrinkles of skin in an effective amount.
[5] At least one compound selected from the group consisting of 1,5-anhydro-D-glucitol and derivatives thereof, for use in promoting collagen production in cells.
[6] At least one compound selected from the group consisting of 1,5-anhydro-D-glucitol and derivatives thereof, for use in preventing and/or improving wrinkles of skin.
[7] Use of at least one compound selected from the group consisting of 1,5-anhydro-D-glucitol and derivatives thereof, for promoting collagen production in cells.
[8] Use of at least one compound selected from the group consisting of 1,5-anhydro-D-glucitol and derivatives thereof, for preventing and/or improving wrinkles of skin.
[9] Use of at least one compound selected from the group consisting of 1,5-anhydro-D-glucitol and derivatives thereof, in the preparation of a composition for promoting collagen production in cells.
[10] Use of at least one compound selected from the group consisting of 1,5-anhydro-D-glucitol and derivatives thereof, in the preparation of a composition for preventing and/or improving wrinkles of skin.

Specifically the present invention provides the following:
1. An external formulation for transdermal administration comprising 1,5-anhydro-D-glucitol or a derivative thereof, represented by the formula (I): 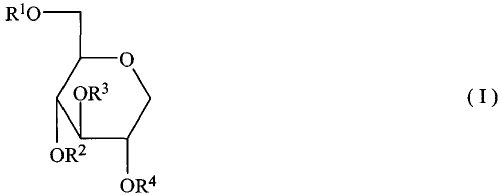 wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time.
2. A non-therapeutic method for promoting collagen production in cells, comprising the step of administering at least one member selected from the group consisting of 1,5-anhydro-D-glucitol and a derivative thereof, represented by the formula (I): 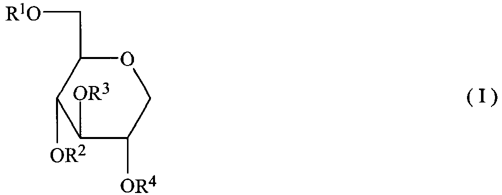 wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time to an individual in need of collagen production promotion in the cells in an effective amount.
3. A non-therapeutic method for preventing and/or improving wrinkles of skin, comprising the step of administering at least one member selected from the group consisting of 1,5-anhydro-D-glucitol and a derivative thereof, represented by the formula (I): 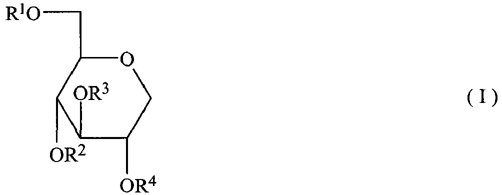 wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time to an individual in need of prevention and/or improvement of wrinkles of skin in an effective amount.
4. Non-therapeutic use of at least one compound selected from the group consisting of 1,5-anhydro-D-glucitol and a derivative thereof, represented by the formula (I): 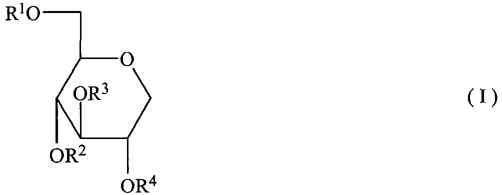 wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time, for promoting collagen production in cells.
5. Non-therapeutic use of at least one compound selected from the group consisting of 1,5-anhydro-D-glucitol and a derivative thereof, represented by the formula (I): 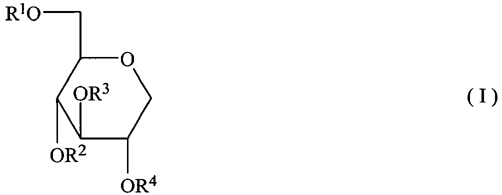
wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time, for preventing and/or improving wrinkles of skin.

### EFFECTS OF THE INVENTION

The collagen production promoter of the present invention exhibits some excellent effects of not only having collagen production promoting functions but also exhibiting moisturizing effects at the same time.

### BRIEF DESCRIPTION OF THE DRAWINGS

[FIG. 1] FIG. 1 is a graph showing the amount of collagen production in human fibroblasts.
[FIG. 2] FIG. 2 is a graph showing the change in water contents of skin surface of human.

### MODES FOR CARRYING OUT THE INVENTION

The collagen production promoter in cells of the present invention (which may be simply referred to as the collagen production promoter of the present invention) is characterized in that the collagen production promoter contains at least one member selected from the group consisting of 1,5-anhydro-D-glucitol (1,5-AG) and derivatives thereof. The phrase "collagen production promotion in cells" as used herein means that when the 1,5-AG and derivatives thereof are allowed to act on cells, the amount of collagen production increases in cells, as compared to a case where they are not allowed to act. For example, the term means that the amount of collagen production in cells is achieved preferably about 110% or more, more preferably about 120% or more, and even more preferably about 130% or more. Here, the cells as used herein are not particularly limited so long as the cells have collagen production ability, and examples of the cells include fibroblasts and keratinocytes.

The 1,5-anhydro-D-glucidol (1,5-AG) has a structure in which 1-position of the D-glucose is reduced, which is one of polyols most abundantly existing in the body. In addition, the derivative of the 1,5-AG is converted to 1,5-AG on skin or in the body, to exhibit the effects of the present invention.

The derivative of the 1,5-AG includes, for example, a compound represented by the formula (I):

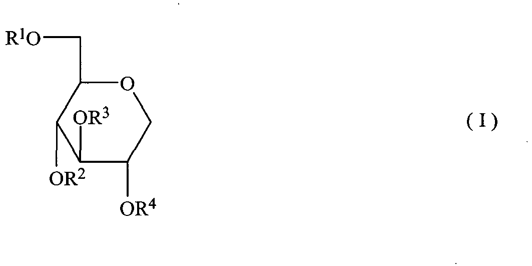

wherein each of R¹, R², R³ and R⁴ is independently a hydrogen atom, a sugar, an amino acid, a vitamin, a vitamin-like active substance, or a fatty acid, with proviso that R¹, R², R³ and R⁴ are not hydrogen atoms at the same time,
as a preferred example.

Each of R¹, R², R³ and R⁴ in the formula (I) is independently a hydrogen atom, a sugar, an amino acid, a vitamin, a vitamin-like active substance, or a fatty acid, with proviso that R¹, R², R³ and R⁴ are not hydrogen atoms at the same time.

The sugar includes monosaccharides which are compounds having from 3 to 7 carbon atoms, such as aldose, ketose, alditol, deoxy sugars, anhydrosugars, aminosugars, inositol, aldonic acid, uronic acid, aldaric acid, carbasugar, thiosugars, iminosugars, and azasugars; and disaccharides and polysaccharides of which constituents are those monosaccharides. Here, the structure of the constituting sugars may be cyclic, such as pyranose or furanose, or linear. Specific examples are monosaccharides such as xylose, lyxose, arabinose, ribose, glucose, allose, galactose, idose, talose, mannose, altrose, erythritol, xylitol, ribitol, arabitol, mannitol, sorbitol, allitol, talitol, galactitol, iditol, myo-inositol, libulose, xylulose, fructose, psicose, tagatose, sorbose, ramnose, fucose, glucuronic acid, glucosamine, and galactosamine; disaccharides such as maltose, lactose, trehalose, isomaltose, sucrose, cellobiose, and nigerose; polysaccharides in which the above monosaccharides and disaccharides are bound in various binding manners such as α1-2 binding, α1-3 binding, α1-4 binding, α1-6 binding, β1-2 binding, β1-3 binding, β1-4 binding, and β1-6 binding.

Among them, monosaccharides such as glucose, galactose, mannose, and glucuronic acid; and disaccharides and polysaccharides of which constituents are those monosaccharides, such as maltose, isomaltose, lactose, and trehalose are preferred, from the viewpoint of safety, biocompatibility and the kinds of enzymes existing in the digestive tract. Here, the derivative having these saccharides is hydrolyzed by a series of enzyme group primarily existing in the digestive tract, such as amylase, glucosidase, mannosidase, galactosidase, fucosidase, and glucuronidase, or hydrolyzed by exposing under physiologically acidic or alkaline conditions on the skin surface or in the digestive tract to convert to 1,5-AG.

The amino acids may be any one of L-form amino acid residues, D-form amino acid residues, mixtures thereof, or derivatives thereof. In addition, the kinds of amino acids may be any one of α-amino acids, β-amino acids, γ-amino acids, and δ-amino acids, and α-amino acid is preferred. Specific examples include glycine, alanine, valine, leucine, isoleucine, serine, threonine, aspartic acid, glutamic acid, asparagine, glutamine, lysine, arginine, cystine, methionine, phenylalanine, tyrosine, tryptophan, histidine, proline, and the like. The derivative having these amino acids is hydrolyzed by peptidase or esterase on skin or in the digestive tract, or hydrolyzed by exposing under physiologically acidic or alkaline conditions on the skin surface or in the digestive tract to convert to 1,5-AG.

Examples of the vitamins and vitamin-like active substances include vitamin A, vitamin B1 (thiamine, thiamine), vitamin B2 (riboflavin), vitamin B5 (pantothenic acid), vitamin B6 (pyridoxine), vitamin B12 (cobalamin), vitamin C (ascorbic acid), vitamin D, vitamin E, vitamin K, folic acid, niacin (vitamin B3, nicotinic acid), vitamin B17 (laetrile, amygdalin), inositol, choline, vitamin F, flavonoid, and the like. The derivative having these vitamins and vitamin-like active substances is hydrolyzed by peptidase or esterase on skin or in the digestive tract, or hydrolyzed by exposing under physiologically acidic or alkaline conditions on the skin surface or in the digestive tract to convert to 1,5-AG.

The fatty acids are preferably a fatty acid having from 2 to 20 carbon atoms, such as octanoic acid, decanoic acid, dodecanoic acid, tetradecanoic acid, hexadecanoic acid, octadecanoic acid, icosanoic acid, or palmitic acid, and more preferably a fatty acid having from 8 to 20 carbon atoms. The derivative having these fatty acids is hydrolyzed by various esterases such as lipase on skin or in the digestive tract, or hydrolyzed by exposing under physiologically acidic or alkaline conditions on the skin surface or in the digestive tract to convert to 1,5-AG.

Here, so long as the sugars, the amino acids, the vitamins, the vitamin-like active substances, or the fatty acids mentioned above are converted to 1,5-AG in live bodies, and preferably on skin surface, these ingredients may be the sugars, the amino acids, the vitamins, the vitamin-like active substances, or the fatty acids themselves, or salts thereof. In addition, the sugars, the amino acids, the vitamins, the vitamin-like active substances, or the fatty acids represented by R¹, R², R³ and R⁴ mean residues bound to the 1,5-AG.

The 1,5-AG and derivatives thereof may be commercially available products, or may be synthesized by any methods known in the art, for example, a method described in Japanese Patent Laid-Open No. 2008-54531, Japanese Unexamined Patent Publication No. 2010-082661, J. Am. Chem. Soc., 72, 4547(1950), or J. Chem. Soc., 214 (1956). Examples of the commercially available products include 1,5-anhydro-D-glucitol (manufactured by Wako Pure Chemical Industries, Ltd.) and the like. The structural confirmation of the synthesized products can be carried out by a known method, for example, NMR, IR, optical rotation, MS, melting point measurement, or the like.

The 1,5-AG and derivatives thereof of the present invention thus obtained can be non-therapeutically used for promoting production of collagen, for example, production of type I collagen, in cells.

The 1,5-AG and derivatives thereof can be used alone or in a combination of two or more kinds. A total content thereof in the collagen production promoter of the present invention is not particularly limited, and a total content is usually from 0.1 to 100% by weight or so.

The form of the collagen production promoter is not particularly limited, so long as at least one member selected from the group consisting of 1,5-AG and derivatives thereof is applicable to a subject substance surface or ingestible in a body, and examples include, for example, powders, liquids, creams, and the like.

Also, the present invention provides a composition containing a collagen production promoter of the present invention. The form of the composition is not particularly limited so long as at least one member selected from the group consisting of 1,5-AG and derivatives thereof can be incorporated into the cells. Examples include, for example, the form as an external formulation composition, from the viewpoint of exhibiting the effects of the 1,5-AG, and the form as an internal formulation composition, from the viewpoint of conveniently ingesting the composition, and the external formulation composition can be preferably used.

The external formulation composition can be provided as a pharmaceutical composition, a quasi-drug composition, or a cosmetic composition.

The form of the external formulation composition includes solid, semisolid, or liquid formulations, for transdermal administration or transmucosal (oral or nasal) administration. Also, the form includes suppositories and the like. The external formulation composition can be in the forms of, for example, liquid formulations such as emulsions and suspensions, such as emulsions and lotions, external tinctures, and liquids for transmucosal administration; ointments such as oily ointments and hydrophilic ointments; adhesive agents for transdermal or transmucosal administration, such as film agents, taping agents, and poultices; spraying agents such as aerosols and sprays; bathing agents, and the like. In addition, in the case of a cosmetic composition, the external formulation composition can be used in any forms of, for example, basic skincare products such as lotions, emulsions, creams, oils, packs, or gels; makeup cosmetics such as foundation, cheek blushes, lipsticks, waxes, and hair tonics; detergents such as soap, shampoos, facial cleansing foams, cleansing lotions, and body detergents; perfumes for skin applications such as colognes, deodorants, and perfumes; bathing agents and the like.

Generally, an internal formulation composition may be provided as a pharmaceutical composition, a quasi-drug composition, a food composition, or a cosmetic composition. Examples of the forms thereof include the form that can be easily blended (for example, powder forms, granular forms), or tablet forms, liquid forms, and the like, from the viewpoint of being conveniently ingestible. Specific examples of the form include pharmaceutical compositions and quasi-drug compositions such as liquids, extracts, elixirs, capsules (hard capsules, soft capsules, etc.), granules, pills, suspensions, emulsions, suppositories, powders, alcoholic agents, tablets, syrups, infusions, decoctions, tinctures, lozenges, aromatic waters, limonades, and fluidextracts; food compositions such as *nimono*, which is a food cooked by boiling or stewing (*tsukudani* (preserved fishes or other foods boiled in soy), *nishime* (vegetables cooked to dryness in soy sauce and water), *nikujaga* (meat and potato stew in soy), etc.), *aemono*, which is chopped fish, shellfish or vegetables dressed with sauce (steamed vegetables dressed with sesame sauce, tofu salad dressed with white sesame sauce, salads, etc.), vinegary salads, fried dishes (*tempura*, *karaage* (Chinese-style chicken deep-fries), deep fries, etc.), steamed dishes (shumai (Chinese dumplings), Japanese steamed egg custard, etc.), *kimpira* (sweetened chopped vegetables cooked in sugar and soy sauce), Chinese steamed or fried dumplings made from a dough of minced pork and vegetable stuffing wrapped with flour sheets, snacks and cakes (*taiyaki* (fish-shaped cake filled with sweetened azuki beans), *obanyaki* (round-shaped batter filled with sweetened azuki beans), *ohagi* (rice ball coated with sweetened azuki beans), *anmochi* (rice cake with sweetened azuki bean paste or azuki bean paste covered with rice cake), *kusamochi* (mugwort-containing rice cake), *dango* (sweet rice cake tiny balls), *manju* (bun filled with azuki bean paste), *senbei* (Japanese rice crackers), *yokan* (azuki bean jelly), *uiro* (sweet rice jelly), *sekihan* (rice cooked with azuki beans), cakes, crepes, cookies, custard puddings, jello, steamed bread, chocolate, caramel, creamy caramel candy, candy, etc.), bread (sandwiches, etc.), liquors, wines, sweet cooking rice wine, soy sauce, juices (orange juice, apple juice, etc.), refreshing beverages, isotonic sports drinks, teas (green tea, barley tea, oolong tea, roasted green tea, etc.), soy-milk, *amazake* (a sweet drink made from fermented rice), Worcestershire sauce, sauce for grilled meat, tomato ketchup, mayonnaise, *tofu* (soybean curd), *miso* (soybean paste), raw noodles, boiled noodles, rehydratable noodles, soup for noodles, salad dressings, *yuzuzu* (yuzu-containing vinegar), *sudachizu* (sudachi-containing vinegar), jam, ham, sausages, meatballs, *kamaboko* (boiled fish paste), *chikuwa* (tube-shaped fish paste cake), deep-fried *surimi*, fish meat sausages, ice cream, cold confectionaries, soft ice cream, milk, yogurt, *hegimochi* (thinly sliced and dried rice cakes), Japanese pickled vegetables, *umeboshi* (Japanese pickled plum), kimchee, *moromi* (main fermenting mash in the production of sake or soy sauce), dried sweet potatoes, dried persimmon, boiled konjak paste, popcorns, cotton candy, *yakiimo* (roasted sweet potatoes), roasted maize, and roasted Japanese chestnuts.

The external composition of the present invention for transdermal administration having the above form can be prepared by properly blending carriers, basal agents, and/or additives and the like, which are ordinarily used in the fields of formulations, cosmetics, foods, etc., according to a conventional method, within the range that would accomplish the object of the present invention, so long as the composition contains at least one member selected from the group consisting of 1,5-AG and derivatives thereof.

Examples of the carriers, the basal agents, and/or the additives and the like, which are used in the field of formulations include excipients (glucose, lactose, sucrose, sodium chloride, starch, calcium carbonate, kaolin, crystalline cellulose, cacao oil, hydrogenated vegetable oil, talc, etc.), binders (distilled water, physiological saline, aqueous ethanol solution, simple syrup, glucose solution, starch solution, gelatin solution, carboxymethyl cellulose, potassium phosphate, polyvinyl pyrrolidone, etc.), disintegrants (sodium alginate, agar, sodium hydrogencarbonate, calcium carbonate, sodium laurylsulfate, stearic acid monoglyceride, starch, lactose, gum arabic powder, gelatin, ethanol, etc.), disintegration inhibitors (sucrose, stearin, cacao oil, hydrogenated oil, etc.), absorption promoters (quaternary ammonium bases, sodium laurylsulfate, etc.), adsorbents (glycerol, starch, lactose, kaolin, bentonite, silicic acid, etc.), and lubricants (purified talc, stearates, polyethylene glycol, etc.). The contents of these ingredients are not particularly limited, and the ingredients can be used in accordance with known techniques.

Examples of the carriers, the basal agents, and/or the additives and the like, which are used in the field of cosmetics include oily ingredients (butyl myristate, isobutyl myristate, butyl palmitate, isobutyl palmitate, butyl stearate, isobutyl stearate, butyl isostearate, cetyl myristate, isostearyl laurate, isostearyl myristate, propylene glycol dicaprate, isostearyl adipate, squalane, liquid paraffin, isoparaffin, beef tallow, lard, mink oil, fish oil, corn oil, cacao oil, almond oil, beeswax, lanolin, reduced lanolin, liquid lanolin, etc.), higher alcohols (lauryl alcohol, myristyl alcohol, cetyl alcohol, stearyl alcohol, oleyl alcohol, behenyl alcohol, hexadecyl alcohol, etc.), fatty acids (caprylic acid, capric acid, undecylenic acid, lauric acid, lauric acid, myristic acid, palmitic acid, stearic acid, oleic acid, linoleic acid, linolenic acid, behenic acid, etc.), ultraviolet absorbents (paraaminobenzoic acid, ethyl dihydroxypropyl paraaminobenzoate, amyl paraaminobenzoate, urocanic acid, sodium hydroxymethoxybenzophenone disulfonate, octyl salicylate, phenyl salicylate, triethanolamine salicylate, etc.), powders and pigments (Red No. 104, Red No. 201, Yellow No. 4, Blue No. 1, Black No. 401, nylon powder, silk powder, chromium oxide, carbon black, aluminum silicate, magnesium myristate, bentonite, zinc palmitate, sericite, calcium carbonate, barium sulfate, etc.), surfactants (fatty acid soaps, alkyl sulfonates, alkylallyl sulfonates, alkylamide sulfates, alkyl phosphates, alkyltrimethylammonium chloride, stearyltrimethylammonium chloride, stearyltrimethylammonium bromide, benzalkonium chloride, carboxybetaine amphoteric surfactants, phosphobetaine amphoteric surfactants, lecithin, saponin, glycerol fatty acid esters, sorbitan fatty acid esters, etc.), polyhydric alcohols and sugars (ethylene glycol, diethylene glycol, propylene glycol, mannitol, erythritol, glucose, sucrose, fructose, trehalose, maltitol, sulfated trehalose, etc.), polymer compounds (acrylate ester/methacrylate ester copolymers, vinyl acetate/crotonic acid copolymers, etc.), antioxidants (sodium sulfite, erythorbic acid, sodium erythorbate, butylhydroxyanisole, lignan, tannin, flavonoids, carotenoids, ascorbyl stearate, parahydroxyanisole, etc.), and solvents (ethanol, purified water, lower alcohols, ethers, N-methylpyrrolidone, fluoro-alcohols, etc.). The contents of these ingredients are not particularly limited, and the ingredients can be used in accordance with known techniques.

Examples of the carriers, the basal agents, and/or the additives and the like, which are used in the field of foods include edible oils (salad oil), glucose, fructose, sucrose, maltose, sorbitol, stevioside, corn syrup, lactose, citric acid, tartaric acid, malic acid, succinic acid, lactic acid, L-ascorbic acid, dl-α-tocopherol, sodium erythorbate, glycerol, propylene glycol, glycerol fatty acid esters, propylene glycol fatty acid esters, gum arabic, sucrose fatty acid esters, sorbitan, casein, gelatin, pectin, agar, vitamin B's, nicotinic acid amide, calcium pantothenate, amino acids, calcium salts, pigments, perfumes, preservatives, and the like. The contents of these ingredients are not particularly limited, and the ingredients can be used in accordance with known techniques.

In addition, the composition may also contain a functional ingredient which is mainly used in the fields of foods. Specific examples include antioxidative substances, reduced ascorbic acid, vitamin E, reduced glutathione, vitamin A derivatives, lycopene, β-cryptoxanthin, astaxanthin, zeaxanthin, fucoxanthin, uric acid, ubiquinone, coenzyme Q10, folic acid, garlic extract, allysine, sesamin, lignans, catechin, isoflavone, chalcone, tannins, flavonoids, coumarin, isocoumarins, blueberry extracts, arbutin, anthocyanin, apple polyphenol, grape seeds extracts, elaidic acid, kojic acid, vitamin A, vitamin B1, vitamin B2, vitamin B6, vitamin B12, vitamin C, vitamin D, vitamin E, vitamin P, choline, niacin, pantothenic acid, calcium folate, EPA, DHA, oligosaccharides, dietary fibers, squalene, soybean lecithin, taurine, Dunaliella, proteins, octacosanol, egg yolk lecithin, linoleic acid, lactoferrin, magnesium, zinc, chromium, serene, potassium, heme iron, oyster extract, chitosan, chitin oligosaccharides, collagen, chondroitin, turmeric, sweetroot, Chinese desert-thorn, cinnamon, hawthorn, ginger, bracket fungus, freshwater-clam extract, *Plantago asiastica*, chamomile, German chamomile, common dandelion (*Taraxacum officinale*), hibiscus, honey, royal jelly, lime, lavender, rose hip, rosemary, sage, Bifidobacteria, wheat germ oil, sesame oil, perilla oil, soybean oil, medium chain fatty acids, agaricus, ginkgo leaf extract, brown rice germ extract, DPA, tiencha (sweet tea), garlic, bee larva, papaya, Pu'er tea, saw palmetto, hyaluronic acid, GABA, shark cartilage, extract of shark cartilage, glucosamine, lecithin, phosphatidylserine, mulberry leaf, soybean extract, jujube, peanut seeds, pearl barley, black vinegar, barley grass, yeast, shiitake mushroom, meat of Japanese pickled plum, amino acids, deep-sea shark extract, noni, oyster meat, strawberry, champignon, iron, zinc, ceramide, silk peptide, Brewer's dried yeast, cocoa, *Kaempferia parviflora*, litchi seed extract, evening primrose extract, black bean extract, coffee bean extract, and the like. The contents of these ingredients are not particularly limited, and the ingredients can be used in accordance with known techniques.

Specific examples of a preparation method include making at least one member selected from the group consisting of 1,5-AG and derivatives thereof directly or in the form of a mixture in the state of dried conditions, various solutions, pastes or the like, blending the mixture with various additives, carriers, basal agents and the like, and powdering, granulating, tableting, dissolving or mixing the obtained mixture, to produce a formulation.

Specific examples of a method for preparing an external formulation include mixing at least one member selected from the group consisting of 1,5-AG and derivatives thereof of the present invention directly or in the form of a mixture, with a solvent or compound having high water solubility such as purified water, ethanol, or glycerol to dissolve, and gradually mixing the solution obtained with a mixture previously prepared from a substance having high fat-solubility such as squalane, jojoba oil, or glycerol monostearate, whereby a lotion and/or cosmetic cream can be prepared.

In addition, the composition of the present invention can contain 1,5-AG and derivatives thereof together with other ingredients which can be used in the same applications as above, for example, a known ingredient having a collagen production promoting action or moisturizing action or the like.

The composition of the present invention is suitably used, for promoting collagen production in cells. For example, the composition can be used in a disease in need of collagen production promoting action for the treatment or prevention of the disease, and specifically, the composition can be used in improvements of injuries due to traumas, such as burns, or abrasions, contusion, cut wounds, incised wounds, or stab wounds. In addition, the composition can be used as a prophylactic agent and/or therapeutic agent for wrinkles of skin caused by ultraviolet ray exposure, drying, aging, or the like. Here, the term "treatment or treating" as used herein is intendedly used to embrace "improvement or improving."

The content of at least one member selected from the group consisting of 1,5-AG and derivatives thereof in the composition of the present invention is not particularly limited, so long as the content is an amount with which the exhibition of the desired effects of the present invention can be obtained, taking into consideration of the dosage form, the method of administration or the like. For example, in a case where the composition is used as an external formulation composition, the content of at least one member selected from the group consisting of 1,5-AG and derivatives thereof is preferably from 0.01 to 10% by weight, more preferably from 0.05 to 7% by weight, and even more preferably from 0.1 to 5% by weight. Here, the phrase "the content of at least one member selected from the group consisting of 1,5-AG and derivatives thereof' as used herein means a total content of 1,5-AG and derivatives thereof contained in the external formulation composition of the present invention.

The composition of the present invention is administered with a suitable method of administration depending upon the form of formulations.

The dose of the composition of the present invention is not a certain level and is properly set by the dosage form, the method of administration, the purposes of use, and age, body weight, and symptoms of a patient to be administered with the composition. For example, in a case of an external formulation, an example of the dose includes preferably from about 0.1 µg to 5 g/day, per about 50 kg body weight of one adult individual. The administration may be a single dose or divided into several doses within one day, within a desired dose range. The administration period is also optional.

The subject to be administered of the present specification is preferably a human in need of the action of promoting collagen production in cells, which may also be pet animals, and the like.

Also, the present invention provides a moisturizing agent, characterized in that the moisturizing agent contains at least one member selected from the group consisting of 1,5-AG and derivatives thereof mentioned above. Since the 1,5-AG and derivatives thereof in the composition of the present invention is a sugar alcohol, the moisturizing action by the sugar alcohol is also exhibited, in addition to the above function of promoting collagen production, so that it can also be suitably used as a moisturizing agent. More specifically, for example, on the surface of the subject substance, moisturizing effects are shown even when used in articles other than live bodies, for those that cause changes such as hydrolysis by physical factors (acidity or alkalinity, water, heat, light or the like), thereby consequently forming 1,5-AG or a 1,5-AG derivative having two or more hydroxyl groups, or a 1,5-AG derivative of a polyol originally having plural hydroxyl groups in the molecule (without causing any chemical transformations on the surface of the article).

The moisturizing agent includes, for example, wetting agents, dry preventives of cosmetics; wetting agents, dry preventives of foods, and the form thereof is not limited. Since the moisturizing agent of the present invention contains 1,5-AG and derivatives thereof from a biological substance, the moisturizing agent has high biological safety.

The content of the moisturizing agent in the cosmetics and foods is not particularly limited, so long as the exhibition of the desired effects of the present invention can be obtained.

The present invention further provides a non-therapeutic method for promoting collagen production in cells, characterized by the use of at least one member selected from the group consisting of 1,5-AG and derivatives thereof mentioned above, or the composition mentioned above. In addition, the present invention provides a non-therapeutic method for preventing and/or improving wrinkles of skin, characterized by the use of at least one member selected from the group consisting of 1,5-AG and derivatives thereof mentioned above, or the composition mentioned above, based on the above function of promoting collagen production,

In these methods, at least one member selected from the group consisting of 1,5-AG and derivatives thereof mentioned above, or the composition mentioned above may be used in an effective amount or more for obtaining the collagen production promotion effects.

### EXAMPLES

The present invention will be explained on the basis of Examples, without intending to limit the scope of the present invention to these Examples and the like. As 1,5-anhydro-D-glucitol (1,5-AG) used in Examples, one synthesized in accordance with a method described in J. Am. Chem. Soc., 72, 4547 (1950) was used.

### Test Example 1

The action of 1,5-AG on the synthesis of type I collagen of dermal fibroblasts is evaluated. Specifically, normal human fibroblasts were spread on a 96-well microplate using Dulbecco's modified eagle medium (DMEM) containing 0.5% fetal bovine serum (FBS). The fibroblasts were cultured at 37°C for 24 hours, and thereafter the medium was exchanged with a 0.5% FBS-containing DMEM containing a given concentration of samples. As a positive control (P.C.), the same treatment was carried out with magnesium ascorbyl phosphate. After the 24-hour culture in the sample-containing medium, the culture supernatant was collected and subjected to ELISA. Also, the amount of protein was measured in accordance with bicinchoninic acid (BCA) method with PIERCE (manufactured by Pierce Biotechnology Inc.).

The culture supernatant and the collagen solutions for calibration curve were each dispensed in each well of high-adsorbent ELISA plate, and allowed to coat at 4°C over one day and night. Thereafter, the coated plate was blocked at 37°C for 1 hour with a 1% bovine serum albumin (BSA) solution. The primary antibody reaction was carried out by diluting Anti-Human Collagen Type I antibody (manufactured by Bio-Rad Laboratories, Inc., Rabbit) with a 0.3% BSA solution, and the dilution was added to each well and allowed to react at 37°C for 1 hour. The secondary antibody reaction was carried out by diluting Histofine MAX-PO(R) (manufactured by NICHIREI, Rabbit) with a phosphate buffer, and the dilution was added to each well, and allowed to react at 37°C for 1 hour. Next, 0.3 mg/mL of phosphate-citrate buffer (0.1 M, pH 4.0) containing 2,2'-azinobis(3-ethylbenzothiazoline-6-sulfonic acid)diammonium salt (ABTS) and a 0.03% hydrogen peroxide was added to each well and allowed to react for 20 minutes, and the absorbance of the reaction mixture was measured at 405 nm with a microplate reader.

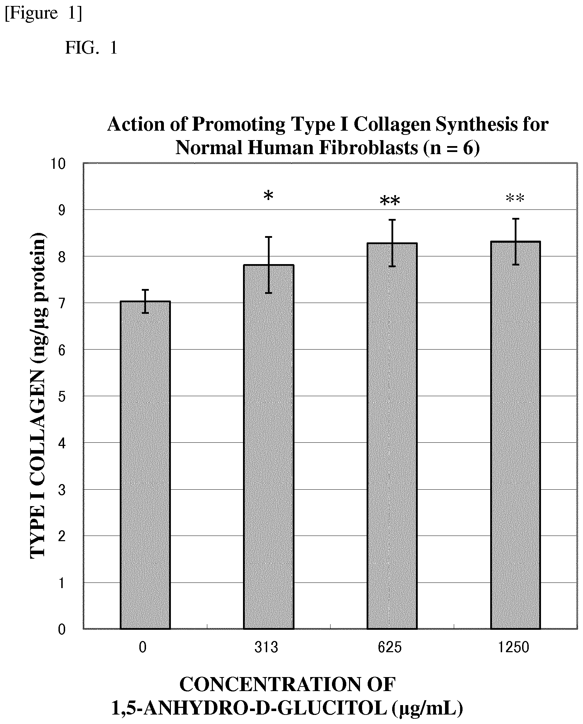

The amount of the collagen in the culture supernatant was calculated from the calibration curve prepared from commercially available collagen. By dividing the amount of collagen in the medium by the amount of proteins in all the cells, the amount of collagen synthesized per unit protein amount was calculated. The amount of collagen synthesized at each concentration was subjected to a significance test using a Student-t test, and a difference with an untreated control was evaluated. The results are shown in Table 1 and FIG. 1.

[Table 1]

**Table 1**

| Action of Promoting Type I Collagen Synthesis for Normal Human Fibroblasts of Samples ( n = 6) | | | | |
|---|---|---|---|---|
| Concentration (µg/mL) | Type I Collagen (ng/µg protein) | | Amount of Protein (µg/well) | |
| | Mean S.D. | p (t-test)¹⁾ | Mean S.D. | p (t-test)¹⁾ |
| 0 | 7.03 ± 0.25 | 1.000 | 9.38 ± 0.21 | 1.000 |
| 313 | 7.81 ± 0.60 | 0.015 * | 9.38 ± 0.44 | 1.000 |
| 625 | 8.28 ± 0.50 | 0.000 * | 9.18 ± 0.46 | 0.355 |
| 1250 | 8.31 ± 0.49 | 0.000 * | 9.09 ± 0.31 | 0.086 |

| | | | | |
|---|---|---|---|---|
| 1) Significance with cells untreated with sample *: Significant increase | | | | |

As is clear from Table 1, the amount of type I collagen synthesized in the normal human fibroblasts significantly increased by the 1,5-AG.

### Test Example 2

The moisturizing effects of the 1,5-AG are evaluated by a short-term use test of human volunteers. Specifically, with 5 males of ages of 26 to 34 as test individuals, an aqueous solution of a sample listed in Table 2 in a given concentration was applied in an amount of 2.5 µL/cm² at a medial part of forearm, and the water content of the skin surface was measured in accordance with the following method.

### < Measurement of Water Content of Skin Surface >

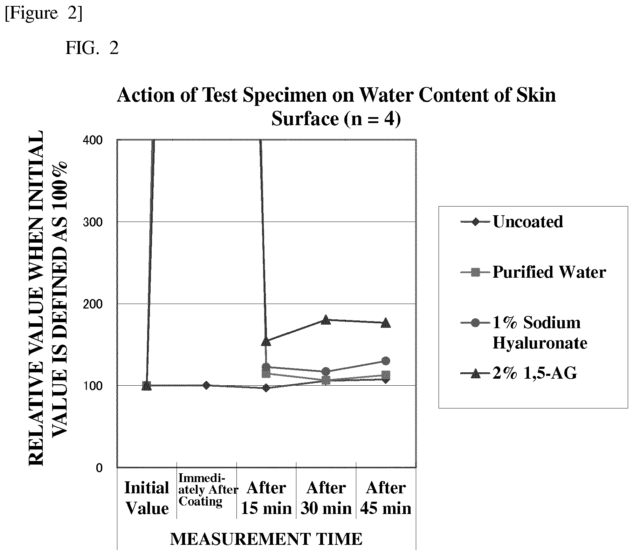

Each test individual washed his medial part of forearm with a given detergent, and was conditioned for 15 minutes in a thermohygrostat (room temperature: 22°C, humidity: 45%). Thereafter, an initial value of the water content of the skin surface was measured with SKICON200 (manufactured by I.B.S. Co., Ltd., corneometer). Next, each of the test samples and purified water were coated according to a coating method. Further, uncoated sites were set. Water contents were measured immediately after the sample coating, after 15 minutes, after 30 minutes, and after 45 minutes. The water content of the uncoated sites was measured matching the measurement time at the sample-coated sites. The change in the water contents of each site to be tested was expressed in a relative value where an initial value was defined as 100%. The difference in the water content of skin surface in each test individual before and after the use of test samples was subjected to a significance test according to a Student-t test. The data for test individuals of which water contents of skin surface of the uncoated sites increased with passage of time were discarded as abnormal values, and the results calculated using the measurement values of four individuals are shown in Table 2 and FIG. 2.

### [Table 2]

**Table 2**

| Action of Test Specimen to Water Content of Skin Surface (n = 4) | | | | | | | | |
|---|---|---|---|---|---|---|---|---|
| Test Specimen | Measurement Time | Mean¹⁾ (%) | | SD | P²⁾ (t-test) | P³⁾ (t-test) | P⁴⁾ (t-test) | P⁵⁾ (t-test) |
| Uncoated | Initial Value | 100.0 | ± | - | - | - | - | - |
| | Immediately after Coating | 100.0 | ± | - | - | - | - | - |
| | After 15 min | 96.7 | ± | 17.2 | 0.727 | - | - | - |
| | After 30 min | 105.6 | ± | 6.2 | 0.170 | - | - | - |
| | After 45 min | 107.3 | ± | 11.7 | 0.303 | - | - | - |
| Purified Water | Initial Value | 100.0 | ± | - | - | - | - | - |
| | Immediately after Coating | 3,381.4 | ± | 3,078.5 | 0.123 | 0.123 | - | - |
| | After 15 min | 114.7 | ± | 6.2 | 0.018* | 0.096 | - | - |
| | After 30 min | 106.3 | ± | 19.1 | 0.558 | 0.940 | - | - |
| | After 45 min | 112.7 | ± | 19.8 | 0.290 | 0.356 | - | - |
| 1% Sodium Hyaluronate | Initial Value | 100.0 | ± | - | - | - | - | - |
| | Immediately after Coating | 3,101.8 | ± | 1,716.3 | 0.040* | 0.040* | 0.758 | - |
| | After 15 min | 122.6 | ± | 15.7 | 0.063 | 0.210 | 0.454 | - |
| | After 30 min | 116.9 | ± | 23.7 | 0.250 | 0.327 | 0.151 | - |
| | After 45 min | 130.1 | ± | 10.3 | 0.010* | 0.015* | 0.077 | - |
| 2% 1,5-AG | Initial Value | 100.0 | ± | - | - | - | - | - |
| | Immediately after Coating | 2,535.4 | ± | 749.6 | 0.007* | 0.007* | 0.638 | 0.566 |
| | After 15 min | 154.2 | ± | 12.3 | 0.003* | 0.015* | 0.004* | 0.025* |
| | After 30 min | 180.3 | ± | 36.2 | 0.021* | 0.020* | 0.004* | 0.003* |
| | After 45 min | 176.7 | ± | 15.8 | 0.002* | 0.000* | 0.003* | 0.005* |

| | | | | | | | | |
|---|---|---|---|---|---|---|---|---|
| 1) Change of water contents at the test sites was expressed in a relative value where the initial value was defined as 100%. 2) Significance with initial value of a test site. 3) Significance with uncoated site at each measurement time. 4) Significance with site coated with purified water at each measurement time. 5) Significance with site coated with 1% sodium hyaluronate at each measurement time. * : Showing a significant increase in water content. | | | | | | | | |

As is clear from Table 2, it was suggested that a test sample of 2% 1,5-AG has an action of increasing the water content of skin surface. This suggests that 1,5-AG shows moisturizing property on the skin surface.

Specific formulations of cosmetics, medicinal formulations, foods, etc. in which the collagen production promoter of the present invention is blended are exemplified hereinbelow. The numerical values at the right end of each formulation example mean % by weight of each content ingredient.

### Formulation Example 1: Milky Lotion

The oily ingredients of the following 1 to 4 are mixed while heating at 70° to 80°C. Next, the water-soluble ingredients of the following 5 to 11 are mixed while heating at 70° to 80°C, and thereto is mixed a mixture of the above oily ingredients while stirring slowly, cautiously not to lower the temperature below 70°C. The mixture is stirred at the same temperature for 12 to 24 hours, and thereafter gradually cooled to room temperature to give an intended milky lotion.

### [Table 3]

**Table 3**

| Formulation Example 1: Milky Lotion | | |
|---|---|---|
| 1 | Squalane | 10.5 |
| 2 | Methylphenyl Polysiloxane | 3.0 |
| 3 | Vitamin E | 0.1 |
| 4 | Sorbitan Monostearate | 25.0 |
| 5 | Glycerol | 5.5 |
| 6 | Methyl Paraoxybenzoate | 0.2 |
| 7 | Carboxyvinyl Polymer | 0.1 |
| 8 | Perfume | 0.05 |
| 9 | Ethanol | 5.2 |
| 10 | 1,5-AG | 1.5 |
| 11 | Purified Water | Bal. |

### [Table 4]

**Table 4**

| Formulation Example 2: Lotion (for Cosmetics and/or Therapy) | | |
|---|---|---|
| 1 | Ethanol | 8.0 |
| 2 | Sorbitan Monostearate Ester | 0.8 |
| 3 | Perfume | 0.2 |
| 4 | Pigment | 0.1 |
| 5 | Citric Acid | 0.1 |
| 6 | Sodium Citrate | 0.2 |
| 7 | Glycerol | 2.0 |
| 8 | 1,5-AG | 1.0 |
| 9 | Purified Water | Bal. |

### [Table 5]

**Table 5**

| Formulation Example 3: Cream | | |
|---|---|---|
| 1 | Squalane | 8.0 |
| 2 | Stearic Acid | 2.0 |
| 3 | Vaseline | 5.0 |
| 4 | Butyl Myristate | 1.1 |
| 5 | Sorbitan Monopalmitate | 1.1 |
| 6 | Pigment | 0.1 |
| 7 | Glycerol | 12.0 |
| 8 | Perfume | 0.1 |
| 9 | Ethyl Paraoxybenzoate | 0.1 |
| 10 | 1,5-AG | 2.0 |
| 11 | Purified Water | Bal. |

### [Table 6]

**Table 6**

| Formulation Example 4: Aqueous Gel | | |
|---|---|---|
| 1 | Octyldodecyl Myristate | 12.0 |
| 2 | Methylphenyl Polysiloxane | 5.2 |
| 3 | Stearic Acid | 3.0 |
| 4 | Glyceryl Monostearate | 2.2 |
| 5 | Methyl Paraoxybenzoate | 0.1 |
| 6 | 1,5-AG | 1.0 |
| 7 | Pigment | 0.1 |
| 8 | Perfume | 0.1 |
| 9 | 1,3-Butylene Glycol | 5.5 |
| 10 | Purified Water | Bal. |

### [Table 7]

**Table 7**

| Formulation Example 5: Facial Cleansing Foam | | |
|---|---|---|
| 1 | Myristic Acid | 15.0 |
| 2 | Lauric Acid | 18.0 |
| 3 | Sorbitan Monostearate | 1.0 |
| 4 | Glycerol | 21.0 |
| 5 | Sodium Hydroxide | 6.2 |
| 6 | Pigment | 0.1 |
| 7 | Perfume | 0.1 |
| 8 | Methyl Paraoxybenzoate | 0.1 |
| 9 | 1,5-AG | 2.5 |
| 10 | Purified Water | Bal. |

### [Table 8]

**Table 8**

| Formulation Example 6: Milky Liquid Foundation | | |
|---|---|---|
| 1 | Stearic Acid | 3.0 |
| 2 | Propylene Glycol Monostearate | 3.0 |
| 3 | Liquid Paraffin | 3.5 |
| 2 | Isopropyl Stearate | 7.0 |
| 4 | Ethyl Parabene | 0.5 |
| 5 | Bentonite | 0.2 |
| 6 | Propylene Glycol | 1.8 |
| 7 | Triethanolamine | 1.0 |
| 8 | Perfume | 0.1 |
| 9 | Pigment | 0.1 |
| 10 | Methyl Paraoxybenzoate | 0.1 |
| 11 | 1,5-AG | 3.5 |
| 12 | Purified Water | Bal. |

### [Table 9]

**Table 9**

| Formulation Example 7: Facial Pack | | |
|---|---|---|
| 1 | Polyvinyl Alcohol | 14.0 |
| 2 | Ethanol | 11.0 |
| 3 | Glycerol | 4.5 |
| 4 | Polyethylene Glycol | 1.8 |
| 5 | Perfume | 0.1 |
| 6 | Methyl Paraoxybenzoate | 0.1 |
| 7 | 1,5-AG | 0.5 |
| 8 | Purified Water | Bal. |

### [Table 10]

**Table 10**

| Formulation Example 8: Hair Tonic | | |
|---|---|---|
| 1 | Ethanol | 55.0 |
| 2 | 1-Menthol | 0.5 |
| 3 | Methyl Paraoxybenzoate | 0.1 |
| 4 | Perfume | 0.1 |
| 5 | 1,3-Butylene Glycol | 3.0 |
| 6 | Glycerol | 2.0 |
| 7 | 1,5-AG | 1.0 |
| 8 | Purified Water | Bal. |

### [Table 11]

**Table 11**

| Formulation Example 9: Ingestible Oral Fluid | | |
|---|---|---|
| 1 | 1,5-Anhydro-D-fructose (1,5-AF) | 5.0 |
| 2 | Citric Acid | 0.1 |
| 3 | 1,5-AG | 5.0 |
| 4 | Leucine | 0.1 |
| 5 | Purified Water | Bal. |

### [Table 12]

**Table 12**

| Formulation Example 10: Granular Ingestible Agent (Medicament) | | |
|---|---|---|
| 1 | Lactose | 28.0 |
| 2 | Cornstarch | 58.0 |
| 3 | Crystalline Cellulose | 9.0 |
| 4 | Polyvinyl Pyrrolidone | 1.0 |
| 5 | 1,5-AG | 4.0 |

### [Table 13]

**Table 13**

| Formulation Example 11: Bathing Agent | | |
|---|---|---|
| 1 | 1,5-AG | 1.0 |
| 2 | Perfume | 0.1 |
| 3 | Pigment | 0.1 |
| 4 | Sodium Hydrogencarbonate | 50.0 |
| 5 | Sodium Sulfate | 48.8 |

### [Table 14]

**Table 14**

| Formulation Example 12: Chewing Gum | | |
|---|---|---|
| 1 | Sucrose | 55.0 |
| 2 | Gum Base | 21.0 |
| 3 | Glucose | 10.0 |
| 4 | 1,5-AG | 0.2 |
| 5 | Pigment | 0.1 |
| 6 | Starch Syrup | 13.6 |
| 7 | Flavor | 0.1 |

### [Table 15]

**Table 15**

| Formulation Example 13: Refreshing Beverage | | |
|---|---|---|
| 1 | Fructose-Glucose Liquid Sugar | 40.0 |
| 2 | Sorbitan Monostearate Ester | 0.5 |
| 3 | Citric Acid | 1.5 |
| 4 | Flavor | 0.5 |
| 5 | 1,5-AG | 0.5 |
| 6 | Purified Water | Bal. |

### [Table 16]

**Table 16**

| Formulation Example 14: Tablet Candy | | |
|---|---|---|
| 1 | Sucrose | 72.0 |
| 2 | Glucose | 18.5 |
| 3 | Sucrose Monostearate Ester | 0.4 |
| 4 | 1,5-AG | 5.0 |
| 5 | Purified Water | 4.1 |

### INDUSTRIAL APPLICABILITY

The collagen production promoter containing 1,5-AG or derivatives thereof of the present invention is suitably used for promoting collagen production in cells, for example, preventing and/or improving wrinkles of skin.

## Claims

1. An external formulation for transdermal administration comprising 1,5-anhydro-D-glucitol or a derivative thereof, represented by the formula (I): 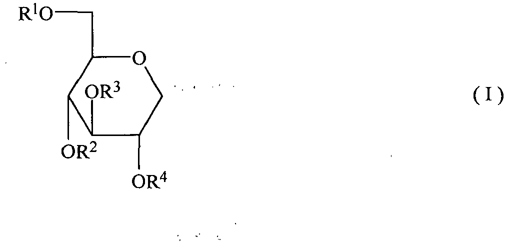 wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time.

2. A non-therapeutic method for promoting collagen production in cells, comprising the step of administering at least one member selected from the group consisting of 1,5-anhydro-D-glucitol and a derivative thereof, represented by the formula (I): 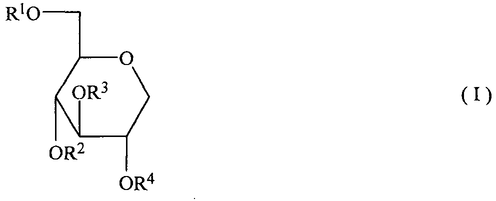 wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time to an individual in need of collagen production promotion in the cells in an effective amount.

3. A non-therapeutic method for preventing and/or improving wrinkles of skin, comprising the step of administering at least one member selected from the group consisting of 1,5-anhydro-D-glucitol and a derivative thereof, represented by the formula (I): 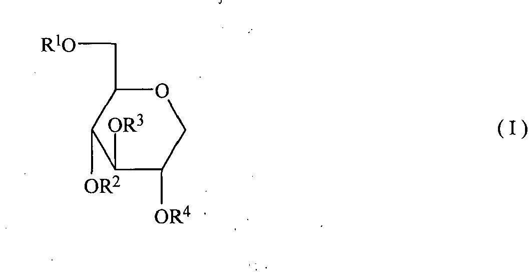 wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time to an individual in need of prevention and/or improvement of wrinkles of skin in an effective amount.

4. Non-therapeutic use of at least one compound selected from the group consisting of 1,5-anhydro-D-glucitol and a derivative thereof, represented by the formula (I): 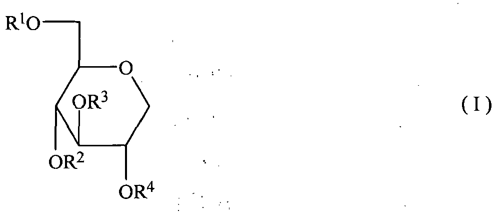 wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time, for promoting collagen production in cells.

5. Non-therapeutic use of at least one compound selected from the group consisting of 1,5-anhydro-D-glucitol and a derivative thereof, represented by the formula (I): 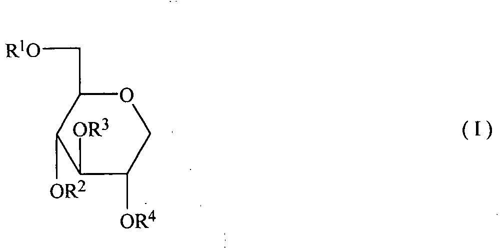 wherein each of R¹, R², R³ and R⁴ is independently a hydrogen atom, a sugar, an amino acid, a vitamin, or a fatty acid, with the proviso that R¹, R², R³ and R⁴ are not hydrogen atoms at the same time, for preventing and/or improving wrinkles of skin.

## Patentansprüche

1. Eine externe Formulierung zur transdermalen Verabreichung, umfassend 1,5-Anhydro-D-glucitol oder ein Derivat davon, dargestellt durch die Formel (I): 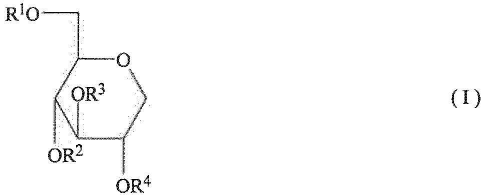 wobei jedes aus R¹, R², R³ und R⁴ unabhängig ein Wasserstoffatom, ein Zucker, eine Aminosäure, ein Vitamin oder eine Fettsäure ist, mit der Maßgabe, dass R¹, R², R³ und R⁴ nicht gleichzeitig Wasserstoffatome sind.

2. Ein nicht-therapeutisches Verfahren zur Förderung der Kollagenproduktion in Zellen, umfassend den Schritt des Verabreichens mindestens eines Mitglieds, ausgewählt aus der Gruppe bestehend aus 1,5-Anhydro-D-glucitol und einem Derivat davon, dargestellt durch die Formel (I): 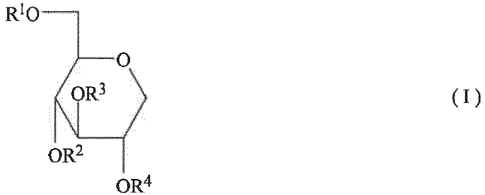 wobei jedes aus R¹, R², R³ und R⁴ unabhängig ein Wasserstoffatom, ein Zucker, eine Aminosäure, ein Vitamin oder eine Fettsäure ist, mit der Maßgabe, dass R¹, R², R³ und R⁴ nicht gleichzeitig Wasserstoffatome sind, an ein Individuum, das eine Förderung der Kollagenproduktion in den Zellen benötigt in einer wirksamen Menge.

3. Ein nicht-therapeutisches Verfahren zur Vorbeugung und/oder Verbesserung von Falten der Haut, umfassend den Schritt des Verabreichens mindestens eines Mitglieds, ausgewählt aus der Gruppe bestehend aus 1,5-Anhydro-D-glucitol und einem Derivat davon, dargestellt durch die Formel (I): 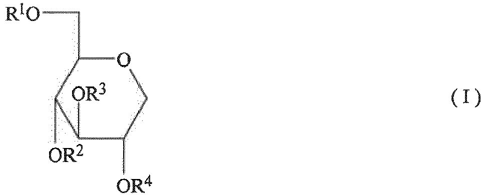 wobei jedes aus R¹, R², R³ und R⁴ unabhängig ein Wasserstoffatom, ein Zucker, eine Aminosäure, ein Vitamin oder eine Fettsäure ist, mit der Maßgabe, dass R¹, R², R³ und R⁴ nicht gleichzeitig Wasserstoffatome sind, an ein Individuum, das eine Vorbeugung und/oder Verbesserung von Falten der Haut benötigt in einer wirksamen Menge.

4. Nicht-therapeutische Verwendung mindestens einer Verbindung, ausgewählt aus der Gruppe bestehend aus 1,5-Anhydro-D-glucitol und einem Derivat davon, dargestellt durch die Formel (I): 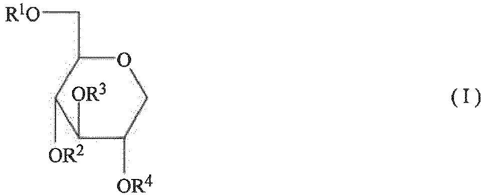 wobei jedes aus R¹, R², R³ und R⁴ unabhängig ein Wasserstoffatom, ein Zucker, eine Aminosäure, ein Vitamin oder eine Fettsäure ist, mit der Maßgabe, dass R¹, R², R³ und R⁴ nicht gleichzeitig Wasserstoffatome sind, zur Förderung der Kollagenproduktion in Zellen.

5. Nicht-therapeutische Verwendung mindestens einer Verbindung, ausgewählt aus der Gruppe bestehend aus 1,5-Anhydro-D-glucitol und einem Derivat davon, dargestellt durch die Formel (I): 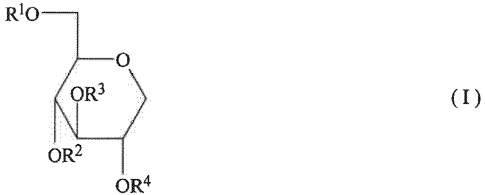 wobei jedes aus R¹, R², R³ und R⁴ unabhängig ein Wasserstoffatom, ein Zucker, eine Aminosäure, ein Vitamin oder eine Fettsäure ist, mit der Maßgabe, dass R¹, R², R³ und R⁴ nicht gleichzeitig Wasserstoffatome sind, zur Vorbeugung und/oder Verbesserung von Falten der Haut.

## Revendications

1. Formulation externe pour une administration transdermique comprenant du 1,5-anhydro-D-glucitol ou un dérivé de celui-ci, représenté par la formule (I) : 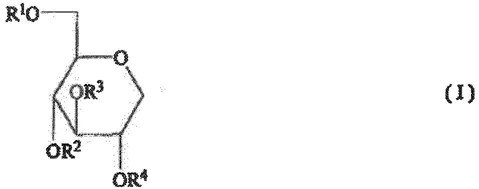 dans lequel chacun de R¹, R², R³ et R⁴ représente indépendamment un atome d'hydrogène, un sucre, un acide aminé, une vitamine, ou un acide gras, à condition que R¹, R², R³ et R⁴ ne représentent pas des atomes d'hydrogène en même temps.

2. Méthode non thérapeutique pour activer la production de collagène dans des cellules, comprenant l'étape d'administration d'au moins un membre choisi dans le groupe constitué du 1,5-anhydro-D-glucitol et un dérivé de celui-ci, représenté par la formule (I) : 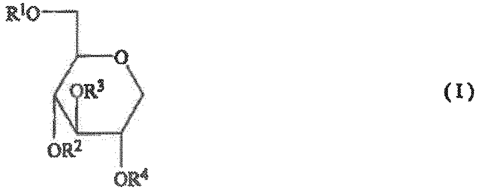 dans lequel chacun de R¹, R², R³ et R⁴ représente indépendamment un atome d'hydrogène, un sucre, un acide aminé, une vitamine, ou un acide gras, à condition que R¹, R², R³ et R⁴ ne représentent pas des atomes d'hydrogène en même temps à un individu ayant besoin d'une activation de la production de collagène dans les cellules dans une quantité efficace.

3. Méthode non thérapeutique pour prévenir et/ou améliorer les rides de la peau, comprenant l'étape d'administration d'au moins un membre choisi dans le groupe constitué du 1,5-anhydro-D-glucitol et un dérivé de celui-ci, représenté par la formule (I) : 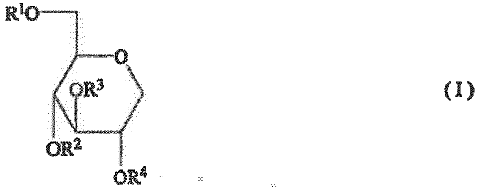 dans lequel chacun de R¹, R², R³ et R⁴ représente indépendamment un atome d'hydrogène, un sucre, un acide aminé, une vitamine, ou un acide gras, à condition que R¹, R², R³ et R⁴ ne représentent pas des atomes d'hydrogène en même temps à un individu ayant besoin d'une prévention et/ou d'une amélioration des rides de la peau dans une quantité efficace.

4. Utilisation non thérapeutique d'au moins un composé choisi dans le groupe constitué du 1,5-anhydro-D-glucitol et un dérivé de celui-ci, représenté par la formule (I) : 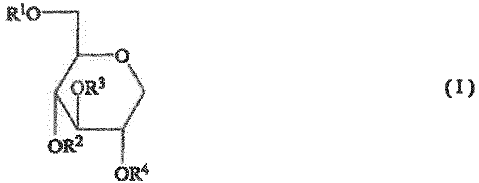 dans lequel chacun de R¹, R², R³ et R⁴ représente indépendamment un atome d'hydrogène, un sucre, un acide aminé, une vitamine, ou un acide gras, à condition que R¹, R², R³ et R⁴ ne représentent pas des atomes d'hydrogène en même temps, pour activer la production de collagène dans des cellules.

5. Utilisation non thérapeutique d'au moins un composé dans le groupe constitué du 1,5-anhydro-D-glucitol et de l'un de ses dérivés, représenté par la formule (I) : 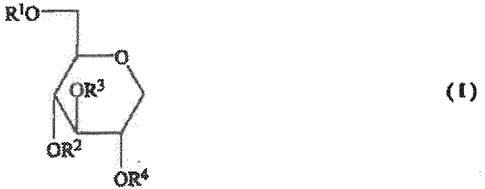 dans lequel chacun de R¹, R², R³ et R⁴ représente indépendamment un atome d'hydrogène, un sucre, un acide aminé, une vitamine, ou un acide gras, à condition que R¹, R², R³ et R⁴ ne représentent pas des atomes d'hydrogène en même temps, pour la prévention et/ou l'amélioration des rides de la peau.
